(19) Europäisches Patentamt
European Patent Office
Office européen des brevets

(11) Veröffentlichungsnummer: **0 169 465**
**B1**

(12) # EUROPÄISCHE PATENTSCHRIFT

(45) Veröffentlichungstag der Patentschrifft: **28.02.90**

(51) Int. Cl. [5]: **A 61 K 31/62**

(21) Anmeldenummer: **85108761.9**

(22) Anmeldetag: **12.07.85**

(54) **Kombinationspräparat aus Pyrimido-pyrimidinen und O-Acetylsalicylsäure bzw. deren pharmakologisch verträglichen Salzen und dessen Verwendung.**

(30) Priorität: **21.07.84 DE 3426961**
**03.05.85 DE 3515874**

(43) Veröffentlichungstag der Anmeldung:
**29.01.86 Patentblatt 86/05**

(45) Bekanntmachung des Hinweises auf die Patenterteilung:
**28.02.90 Patentblatt 90/09**

(84) Bennante Vertragsstaaten:
**AT BE CH DE FR GB IT LI LU NL SE**

(56) Entgegenhaltungen:
**FR-A-2 368 272**
**FR-A-2 390 959**

(73) Patentinhaber: **HOECHST AKTIENGESELLSCHAFT**
**Postfach 80 03 20**
**D-6230 Frankfurt am Main 80 (DE)**

(72) Erfinder: **Weithmann, Klaus Ulrich, Dr.**
**Am Domherrenwald 18**
**D-6238 Hofheim am Taunus (DE)**
Erfinder: **Seiffge, Dirk, Dr.**
**Hauptstrasse 2**
**D-6309 Münzenberg (DE)**

EP 0 169 465 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

O-Acetylsalicylsäure ist als Hemmstoff der Aggregation menschlicher Blutplättchen bekannt (z. B. Br. J. Clin. Pharmac. 7 (1979) 283), und es wurde berichtet, daß sie hinsichtlich der Vermeidung von Thrombosen und Schlaganfällen wertvolle therapeutische Effekte zeigen kann (Blood 52 (1978) 1073, N. Engl. J. Med. 299 (1978) 53). Als Wirkungsmechanismus ist beschrieben, daß Acetylsalicylsäure das in den Blutplättchen lokalisierte Enzym Cyclooxygenase hemmt (J. Clin. Invest. 56 (1975) 624) und somit die Biosynthese des die Aggregation fördernden Thromboxan A2 inhibiert wird. Allerdings kann Acetylsalicylsäure auch die in der Gefäßwand befindliche Cyclooxygenase hemmen und damit die Synthese des die Aggregation hemmenden Prostazyklins. Da die Hemmung der vaskulären Cyclooxygenase aber erst bei höheren Dosen Acetylsalicylsäure zu beobachten ist (Pharmacol. Research Commun. 10 (1978) 759), wird konsequenterweise empfohlen, mit niedrigen Acetylsalicylsäure-Dosierungen eine antithrombotische Wirkung zu erreichen (Lancet, iii (1979) 1213, Prostaglandins and Medicine 4 (1980) 439). Es ist aber auch beschrieben worden, daß die antithrombotische Wirkung von Acetylsalicylsäure mit steigender Dosierung zunimmt und eine optimale Wirkung unter Bedingungen erreicht wird, bei denen sowohl die Prostazyklin- als auch die Thromboxan-Biosynthese weitgehend gehemmt wird (Prostaglandins, Leukotrienes and Medicine 12 (1983) 235).

Pyrimido-pyrimidine (Formel I, siehe Formelblatt), worunter im folgenden insbesondere Dipyridamol (2,6-Bis-(diäthanol - amino)-4,8-dipiperidino-(5,4-d)-pyrimidin) und Mopidamol (2,6-Bis-(diäthanolamino)-8-piperidino-(5,4-d)-pyrimidin) zu verstehen sind (siehe Formelblatt) werden in der Klinik ebenfalls als antithrombotische und antiaggregatorische Medikamente (Platelets and Thrombosis, S. 175, Mills and Poreti (Hrsg.) Academic Press, New York (1977)) bzw. zur Inhibierung von Metastasen eingesetzt. Klinisch besonders wertvoll ist offenbar auch die Förderung der Sauerstoffversorgung ischämischer Bezirke in den Herzkranzgefäßen beim Infarkt (Herz-Kreislauf 5 (1973) 519 - 524). Dipyridamol ist als Stimulator von Prostazyklin beschrieben worden (Arch. Int. Pharmacodyn. *256* (1982) 327). Für Kombinationen aus Dipyridamol und Acetylsalicylsäure sind überadditive Wirkungen auf die induzierte Thrombocytenaggregation beschrieben (FR-PS-2 368 272) und entsprechende therapeutische Zubereitungen vorgeschlagen worden. Da aus den beiden Komponenten keine stabilen einheitlichen Mischungen erhalten werden können, wurde in der genannten FR-PS vorgeschlagen, die Komponenten in Schichttabletten bzw. Mantel-Kern-Tabletten räumlich voneinander zu trennen.

Ein Kombinationspräparat, das 330 mg Acetylsalicylsäure und 75 mg Dipyridamol enthält, wird bereits medizinisch angewendet (Asasantin®, Fa. Dr. Karl Thomae GmbH, Biberach). Auch 1 g Acetylsalicylsäure kombiniert mit 75 mg Dipyridamol ist bereits angewendet worden (Europ. J. Clin. Pharmacol. 22 (1982) 309 - 314). Die Kombination anderer Pyrimido-pyrimidine mit Acetylsalicylsäure ist ebenfalls bekannt, aber nur im Verhältnis 0,5 bis 0,33 oder noch kleiner (FR-PS-2 390 959).

Wie in Tab. I gezeigt wird, können mit der Kombination aus 5 mg/kg KG Dipyridamol und 22 mg/kg KG (Körpergewicht) Acetylsalicylsäure höhere antithrombotische Wirkungen erzielt werden als durch Addition der Wirkung der Einzelsubstanzen berechnet werden kann, d. h. diese Kombination zeigt nicht nur die bereits oben erwähnten überadditiven antiaggregatorischen Effekte, sondern auch einen überadditiven antithrombotischen Effekt. Wie unten ausführlicher beschrieben wird, kann die medizinische Anwendung von Acetylsalicylsäure auch unerwünschte Nebenwirkungen, z. B gastrointestinale Irritationen, hervorrufen. Man müßte daher bestrebt sein, den Acetylsalicylsäure-Anteil in der Kombination zu erniedrigen, d. h. das Verhältnis Pyrimido-pyrimidin zu Acetylsalicylsäure großer als 0,5 zu wählen. Wird nun der Acetylsalicylsäure-Anteil in der genannten Kombination der Tabelle 1 bei gleichzeitiger Verabreichung von 22 mg/kg auf 5 mg reduziert, dann läßt sich kein signifikanter antithrombotischer Effekt mehr nachweisen, d. h. die aus Gründen der medizinischen Verträglichkeit erwünschte Verminderung des Acetylsalicylsäure-Anteils führt zu einem Präparat ohne medizinischen Effekt.

Es wurde nun überraschend gefunden, daß eine mit zeitlichem Abstand aufeinanderfolgende Anwendung von

A) Pyrimido-pyrimidinen der Formel I (siehe Formelblatt und Patentanspruch 1) bzw. deren wirksamen Metaboliten und/oder Salzen der Verbindungen bzw. Metabolite einerseits und

B) O-Acetylsalicylsäre bzw. deren pharmazeutisch verträglichen Salzen andererseits in einer bestimmten Reihenfolge eine überaus starke Verbesserung der Therapie von Krankheiten ermöglicht, die durch gestörte Blutfunktionen bzw. gestörte Blutbestandteile verursacht bzw. gekennzeichnet sind. Die zeitlich abgestufte Anwendung der Komponente A, die erst nach 20 bis 90 Minuten von der Anwendung der Acetylsalicylsäure bzw. ihres Salzes gefolgt wird, führt zu viel stärkeren Effekten als der berechneten Summe der Wirkungen der Einzelkomponenten entspricht, aber auch zu stärkeren Effekten als wenn die beiden Einzelsubstanzen gleichzeitig oder in umgekehrter Reihenfolge verabfolgt werden. Dies ist umso überraschender, als die gleichzeitige Verabreichung von Pyrimido-pyrimidinen, wie Dipyridamol, und Acetylsalicylsäure nur zu einer unwesentlich stärkeren Wirkung führt als man sie bei der Einzelgabe der Acetylsalicylsäure erhält (vgl. unten Tabelle 1).

Als Pyrimido-pyrimidine kommen insbesondere Dipyridamol (s. Formelblatt Formel I a) und/oder Mopidamol (Formel I b) in Betracht, ferner aber auch z. B. die Verbindungen I c und I d sowie Analoga, in denen ein oder beide $-CH_2-CH_2-OH$-Reste durch $-CH_2-CH(CH_3)-OH$ ersetzt sind oder der Piperidinrest durch den Morpholinrest ersetzt ist.

Gegenstand der Erfindung sind nun Kombinationspräparate, enthaltend A) Pyrimido-pyrimidine der Formel I (s. Patentanspruch 1), insbesondere Dipyridamol und/oder Mopidamol bzw. deren wirksame Metabolite und/oder Salze der Verbindungen bzw. Metabolite und B) O-Acetylsalicylsäure bzw. deren pharmazeutsich verträgliche Salze, wobei das Gewichts-Verhältnis der Komponente A) zur Komponente B) größer als 0,5 ist, mit oder ohne C) einen pharmazeutischen Träger zur zeitlich abgestuften Anwendung in der Therapie von Krankheiten, die durch

gestörte Blutfunktionen bzw. Blutbestandteile, insbesondere Thrombocyten bzw. Erythrocyten verursacht bzw. gekennzeichnet sind, derart, daß die Komponente A) zuerst freigesetzt (bioverfügbar) wird.

Es ist überraschend, daß sich der erfindungsgemäß erhaltene medizinische Effekt nur erzielen läßt, wenn der relative Acetylsalicylsäure-Anteil bei der zeitlich aufeinanderfolgenden (sequentiellen bzw. konsekutiven) Gabe so klein ist, daß ein Gewichtsverhältnis von Pyrimido-pyrimidin zu Acetylsalicylsäure von 0,5 nicht unterschritten wird. Wird das Verhältnis der Komponente A) zur Acetylsalicylsäure-Komponente B) kleiner als 0,5, d. h. steigt der Acetylsalicylsäure-Anteil an, dann läßt sich hinsichtlich der antithrombotischen Wirkung kein signifikanter Unterschied zwischen gleichzeitiger und erfindungsgemäßer aufeinanderfolgender Gabe nachweisen. Es ist auch überraschend, daß mit steigendem Acetylsalicylsäure-Anteil keine bessere antithrombotische Wirkung erreicht werden kann, als bei der erfindungsgemäßen Anwendung der Pyrimido-pyrimidin Komponenten in Kombination mit wesentlich geringeren Mengen an Acetylsalicylsäure, nämlich im Verhältnis von 0,5 oder mehr, z. B. 0,8 oder 1.

Die erfindungsgemäßen Mittel eignen sich aufgrund ihrer überadditiven Effekte also zur antithrombotischen, schmerzstillenden, antiaggregatorischen und cystostatischen, insbesondere Metastasen inhibierenden, Therapie bzw. Prophyllaxe. Gegenstand der Erfindung ist daher auch die Herstellung des Kombinationspräparates und seine Verwendung in der Human- bzw. Veterinärmedizin. Durch die erfindungsgemäßen Kombinationspräparate wird es möglich, daß die Pyrimido-pyrimidin-Komponente zuerst, d. h. vor der Acetylsalicylsäure freigesetzt (bioverfügbar) wird.

Es ist ein überraschender Vorteil, daß wegen des überadditiven Effektes bei der aufeinanderfolgenden Anwendung die anzuwendenden Mengen an Pyrimido-pyrimidin und Acetylsalicylsäure-Komponente auf solche Mengen reduziert werden können, die bei ihrer alleinigen Verabreichung eine nur minimale pharmakologische Wirkung zeigen, so daß gleichzeitig Nebenwirkungen, die von hohen Dosen dieser Arzneimittel hervorgerufen werden, verringert werden. Dies ist deswegen von großer Bedeutung, weil bekanntlich Acetylsalicylsäure in den üblichen Dosierungen unerwünschte Nebenwirkungen (z. B. British Journal of Clinical Pharmacology 1980, 10 Suppl. 2 und International Meeting on Side Effects of Antiinflammatory, Analgesic Drugs, Verona, 13.-15.9.82, Abstracts) wie Asthma, allergische Urtikaria, analgetische Nephropathie, Reye Syndrom, sowie Magen- und Darmulcera hervorrufen kann. Auch die Pyrimido-pyrimidine, wie Dipyridamol, können unerwünschte Nebenwirkungen zeigen (siehe z. B. Goodman and Gilman's The Pharmacological Basis of Therapeutics, 6. Auflage, New York, 1980). Durch das erfindungsgemäße Kombinationspräparat kann nun überraschenderweise die erforderliche Dosierung an Acetylsalicylsäure an Mensch und Tier drastisch reduziert werden und auch die Menge an Dipyridamol bzw. Pyrimido-pyrimidinen so daß die allgemeine toxikologische Verträglichkeit noch weiter verbessert wird.

Geeignete pharmakologisch verträgliche Salze der Acetylsalicylsäure sind solche mit pharmakologisch verträglichen Metallkationen, Ammonium, Aminkationen oder quaternären Ammoniumkationen. Bevorzugt sind solche Alkalimetalle, wie Lithium, Natrium und Kalium, und der Erdalkalimetalle, wie Magnesium und Calcium, obgleich auch kationische Formen anderer Metalle, wie Aluminium, Zink und Eisen verwendet werden können.

Pharmakologisch verträgliche Aminkationen sind z. B. solche von primären, sekundären oder tertiären Aminen wie der Alkylamine, z. B. Methyl-, Dimethyl-, Trimethyl-, Äthyl-, Dibutyl-, Triisopropyl-, N-Methylhexyl-, Benzyl-, β-Phenyl-äthylamin, Äthylendiamin, Diäthylentriamin, Piperidin, Morpholin, Piperazin, Mono-, Di- und Triäthanolamin, Äthyldiäthanolamin, N-Butyläthanolamin und dergleichen. Geeignete Aminsalze sind auch die basischen Aminsalze des Lysins und des Arginins. Geeignete pharmakologisch verträgliche quaternäre Ammoniumkationen sind z. B. das Tetramethylammonium, Tetraäthylammonium und das Benzyltrimethylammonium.

Auch Dipyridamol bzw. Pyrimido-pyrimidine können in Form von Salzen vorliegen, z. B. als Chlorid oder Fluorid. Die Pyrimido-pyrimidin-Komponente einerseits und die Acetylsalicylsäure-Komponente andererseits können zur Erzielung der überadditiven Wirkung auch gleichzeitig verabreicht werden, wobei aber eine Verabreichung in Dosiseinheiten in getrennter Form notwendig ist, wenngleich die Komponenten auch in Gemischen in einer geeigneten Form, die eine zeitlich aufeinanderfolgende Anwendung ermöglichen, verabreicht werden können, z. B. in der Weise, daß die Acetylsalicylsäure in einer nur sehr langsam resorbierbaren Form, z. B. als Aluminiumsalz angewendet wird.

Die Dosiseinheiten können in Form von festen Arzneiformen, wie Kapseln (einschließlich Mikrokapseln, die pharmazeutische Träger enthalten oder nicht), Tabletten (einschließlich Dragees und Pillen) oder Zäpfchen vorliegen, wobei bei Verwendung von Kapseln das Kapselmaterial die Funktion des Trägers wahrnehmen und der Inhalt z. B. als Pulver, Gel, Emulsion, Dispersion oder Lösung vorliegen kann. Dabei ist darauf zu achten, daß dafür Sorge getragen wird, daß die Freisetzung der Wirkstoffe zeitlich abgestuft (sequentiell) erfolgt. Besonders vorteilhaft und einfach ist es, orale (perorale) Formulierungen mit den beiden Wirkstoffen herzustellen, die die berechneten Mengen der Wirkstoffe zusammen mit jedem gewünschten pharmazeutischen Träger enthalten. Auch eine entsprechende Formulierung (Zäpfchen) für die rektale Therapie kann hergestellt und angewandt werden. Ebenso ist die transdermale und parenterale (intraperitoneale, intravenöse, subkutane, intramuskuläre) Injektion von Lösungen z. B. mittels geeigneter Mehrkammer-Injektionseinheiten möglich.

Die sequentielle Freigabe (Bioverfügbarkeit) der Wirkstoffe wird generell erreicht, wenn man in üblicher Weise, z. B. in Tabletten, Pillen oder Granulatkörpern, Acetylsalicylsäure oder deren pharmazeutisch verträgliche Salze in retardierter Form mit dem gewünschten Pyrimido-pyrimidin, wie Dipyridamol, kombiniert.

So können beispielsweise Mantel/Kern-Tabletten entsprechend dem Stand der Technik hergestellt werden. Zwar wird in der FR-PS-2 368 272 neben anderen Möglichkeiten schon erwähnt (Anspruch 7), daß das Dipyridamol in der äßeren Schicht und die Acetylsalicylsäure in der inneren Schicht der Tablette enthalten sein könne. In der Beschreibung und den Ansprüchen 6, 8 und 9 wird auch eine Anzahl von Kombinationsmöglichkeiten aufgeführt, nach denen das Dipyridamol entweder in der inneren Schicht enthalten und von einer darmsaftlöslichen Schicht und die Acetylsalicylsäure von einer magensaftlöslichen Schicht umgeben sein soll, oder wonach sowohl das Dipyridamol als auch die Acetylsalicylsäure von einer magensaftlöslichen Schicht umgeben sind. Mit derartigen pharmazeutischen Zubereitungen läßt sich die erfindungsgemäße sequentielle Freisetzung der Wirkstoffe aber keinesfalls erreichen.

Ein Hinweis auf eine Kombination, in der das Dipyridamol in einer äßeren löslicheren, insbesondere magensaftlöslichen Schicht enthalten ist, und der Kern von einer Acetylsalicylsäure-Tablette gebildet wird, die von einer unlöslicheren, insbesondere magensaftunlöslichen Schicht umgeben ist, findet sich in der FR-PS-2 368 272 nicht. Gerade mit einer solchen Kombination können aber die vorteilhaften erfindungsgemäßen medizinischen Effekte erhalten werden. Ebenso bezieht sich die Erfindung auch auf Mehrschichttabletten, bei denen die Acetylsalicylsäure enthaltende Schicht eine verzögerte Freisetzung der Acetylsalicylsäure gewährleistet. Ganz besonders vorteilhaft ist es allerdings, wenn die Acetylsalicylsäure nicht in der magensäureresistenten Tablettenform vorliegt, sondern in Form von magensäureresistenten Mikrokapseln (Granulate oder Kristalle).

Es ist nämlich bekannt, daß die Anwendung von magensäureunlöslichen Acetylsalicylsäuretabletten problematisch ist (siehe Biopharmaceutics and Relevant Pharmacokinetics. Enteric Coatings, Drug Intelligence Publications. 1. Auflage, 1971, Seite 158 - 165 sowie Aust. N. Z. J. Med. *6* (1976) 45 - 50 sowie Arthritis and Rheumatism *22* (9): 1034 - 1037, September, 1979). Neben erhöhten fäkalen Blutausscheidungen der damit behandelten Patienten ist die zeitlich stark streuende Bioverfügbarkeit der Acetylsalicylsäure zu nennen. Erfindungsgemäß muß ein Zeitabstand von 15 Minuten bis zu 2 Stunden, vorzugsweise 30 bis zu 90 Minuten und insbesondere 40 bis 70 Minuten zwischen der Freisetzung der Pyrimido-pyrimidin-Komponenten und der Acetylsalicylsäure-Komponente liegen. Die Resorption von Acetylsalicylsäure aus magensäureresistenten Tabletten im Darm erfolgt aber mit einer großen interindividuellen zeitlichen Streubreite zwischen den behandelten Patienten. Im manchen Fällen erfolgt die Resorption erst nach 12 Stunden. (Current Therapeutic Research 36 (1984) 811 - 818, JAMA 193 (1965) 93 - 98 sowie Pharmacology 30 (1985) 40 - 44). Darüberhinaus können sich derartige Tabletten im Magen-Darm-Trakt akkumulieren und dadurch erhebliche gastrointestinale Nebenwirkungen hervorrufen (J. of Rheum. 11 (1984) 250 - 251).

Im Gegensatz dazu haben kleinkörnige magensäureunlösliche Granulate bekanntlich verschiedene Vorteile (siehe z. B. Current Therapeutic Research 36 (1984) 811 - 818, European J. Clin. Pharmacol. 14 (1978) 351 - 355 und Eur. J. Clin. Pharmacol. 27 (1984), 74). Bei Granulaten beträgt die Absorptionsverzögerung 0,5 - 1 Stunde, bei gleichzeitig wesentlich kleineren interindividuellen Schwankungen zwischen den Patienten. Ein Zeitabstand von 0,5 - 1 Stunde ist hervorragend für die Erzielung der erfindungsgemäß erhältlichen therapeutischen Effekte geeignet. Ein weiterer Vorteil ist es, daß mit magensäureresistenten Granulaten der Zeitabstand zwischen den erfindungsgemäßen Wirkstofffreigaben sehr zuverlässig eingehalten wird. Da gerade die erfindungsgemäß erhältlichen überadditiven antithrombotischen Effekte vom Zeitintervall zwischen den beiden Wirkstoffgaben bzw. der Freisetzung abhängig sind, eignen sich insbesondere magensäureresistente Granulate für die Herstellung der erfindungsgemäßen Kombinationspräparate. Derartige Acetylsalicylsäure-Granulate können sowohl als Gemisch mit der Pyrimido-pyrimidin-Komponente in einem inerten pharmazeutischen Träger, wie in einer Kapsel, als auch in zwei verschiedenen Kompartimenten der Dosiseinheit vorliegen. Es können aber auch Zubereitungen Anwendung finden, die Acetylsalicylsäure in einer Form enthalten, die eine langsame Freisetzung in Magensäure zuläßt. Zum Beispiel kann Acetylsalicylsäure an Ionenaustauscher oder geeignete Metallionen, wie Aluminium, gebunden, oder an Retardierungsmaterial adsorbiert bzw. in Retardierungsmaterial (z. B. auf Zellulose-oder Polystyrolharzbasis) eingeschlossen sein, welches sich beispielweise im Innern einer Manteltablette befindet, deren Mantel Pyrimido-pyrimidin enthält.

Die erfindungsgemäßen pharmazeutischen Zubereitungen können nach üblichen Verfahren wie Preß-, Tauch- oder Wirbelbettverfahren oder Kesseldragierung, hergestellt werden und enthalten Trägermittel und andere übliche Hilfsstoffe, wie Stärke, z. B. Kartoffel-, Mais- oder Weizenstärke, Zellulose bzw. deren Derivate, insbesondere mikrokristalline Zellulose, Siliziumdioxid, verschiedene Zucker, wie Milchzucker, Magnesiumcarbonat und/oder Kalziumphosphate. Die Dragierlösung besteht gewöhnlich aus Zucker und/oder Stärkesirup, und enthält Gelatine, Gummi arabicum, Polyvinylpyrrolidon, synthetische Zelluloseester, oberflächenaktive Substanzen, Weichmacher, und/oder Pigmente und ähnlichen Zusätzen entsprechend dem Stand der Technik. Zur Herstellung der Arzneiformen kann jedes der üblichen Fließregulierungs-, Schmier- bzw. Gleitmittel wie Magnesiumstearat und Trennmittel verwendet werden.

Das Gewichtsverhältnis der Pyrimido-pyrimidin-Komponente zu Acetylsalicylsäure kann in gewissen Grenzen schwanken, wie weiter unten aufgeführt wird. Das optimale Verhältnis kann beispielsweise mit Hilfe der unten beschriebenen experimentellen Verfahren ermittelt werden. Durch die erfindungsgemäße Anwendung wird es wegen des überadditiven Effektes möglich, die Dosierung der Einzelkomponenten entscheidend zu erniedrigen, und zwar weit unter die Dosierungen, die man zur Erzielung des gleichen Effektes mit Einzelgaben Acetylsalicylsäure oder Pyrimido-pyrimidin oder mit der kombinierten gleichzeitigen Verabreichung von Acetylsalicylsäure und Pyrimido-pyrimidin benötigen würde. Durch die erfindungsgemäße Erniedrigung der Dosierungen werden die bekannten Unverträglichkeitsreaktionen der Pyrimido-pyrimidine sowie von Acetylsalicylsäure ausgeschaltet oder wenigstens deutlich reduziert. Insbesondere wird es durch die Erfindung möglich, das Gewichtsverhältnis von Dipyridamol zu Acetylsalicylsäure größer als 0,5 einzustellen. Wie unten gezeigt wird, kann nämlich durch die Erhöhung des relativen Acetylsalicylsäuregewichtsanteils in der erfindungsgemäßen Kombination auf Werte über 2 (entsprechend einem Verhältnis der Komponente A) zu B) von unter 0,5) der therapeutische Effekt nicht mehr gesteigert werden. Zur Erzielung des erfindungsgemäß erhältlichen überadditiven Effektes ist es also völlig unnötig, und im Hinblick auf die Nebenwirkungen der Acetylsalicylsäure sogar schädlich, den relativen Acetylsalicylsäure-Anteil auf

das 2- bis 3-fache und mehr zu erhöhen, wie das in der FR-PS-2 390 959 vorgeschlagen wird. Demgegenüber beträgt nach der vorliegenden Erfindung das Gewichtsverhältnis Pyrimido-pyrimidin zu Acetylsalicylsäure mehr als 0,5 und bis 30, vorzugsweise von 0,6 bis 10, und insbesondere von 0,6 bis 3 liegt.

Auch der optimale Zeitabstand zwischen der Anwendung der Pyrimido-pyrimidin-Komponente und der Acetylsalicylsäure-Komponente bzw. die optimale Freisetzungsrate aus den galenischen Zubereitungen können durch Anwendung der beschriebenen experimentellen Verfahren ermittelt werden.

Die anzuwendende Dosierung ist selbstverständlich abhängig von verschiedenen Faktoren, wie dem zu behandelnden Lebewesen (d. h. Mensch oder Tier, Alter, Gewicht, allgemeiner Gesundheitszustand), der Schwere der Symptome, der zu behandelnden Erkrankung, (falls vorhanden) der Art der begleitenden Behandlung mit anderen Arzneimitteln, der Häfigkeit der Behandlung usw. Die Dosierungen werden im allgemeinen bis zu fünfmal pro Tag und vorzugsweise einmal bis dreimal pro Tag verabreicht. Die Menge der Bestandteile soll innerhalb des für das zu behandelnde Lebewesen verträglichen, wirksamen Dosierungsbereichs liegen.

Z. B. beträgt die bevorzugte Dosis Acetylsalicylsäure beim Menschen bei alleiniger Verabreichung zwei- bis dreimal täglich 500 bis 2000, insbesondere 1000 mg. Die bevorzugte Dosis Dipyridamol beträgt beim Menschen bei alleiniger Verabreichung zwei- bis dreimal täglich 10 bis 150, insbesondere 25 mg bis 80 mg. Eine geeignete Therapie besteht somit z. B. in der Verabreichung von einer, zwei oder mehr, vorzugsweise 3 bis 8 Einzeldosierungen der erfindungsgemäßen Kombinationspräparate von je 10 bis 150, vorzugsweise mindestens 25 und insbesondere bis 75 mg Dipyridamol und 10 bis 300, z. B. bis 280, vorzugsweise bis 80 mg Acetylsalicylsäure, wobei die Menge naturgemäß von der Zahl der Einzeldosierungen und auch der zu behandelnden Krankheit abhängig ist und eine Einzeldosierung z. B. auch aus mehreren, gleichzeitig verabreichten Tabletten bestehen kann. Dies gilt auch für die erfindungsgemäße Kombination von Mopidamol, von dem im allgemeinen 100 - 600, vorzugsweise 200 - 250 mg zusammen mit 10 bis unter 1200, vorzugsweise 100 bis 300 mg oder 500 mg Acetylsalicylsäure verabreicht werden. Aus diesen Angaben können auch leicht die Mengen in den Dreier-Kombinationen aus Acetylsalicylsäure und zwei Pyrimido-pyrimidinen errechnet werden.

Die erfindungsgemäßen Mittel können in gleicher Weise wie bekannte antithrombotische und die Blutplättchenaggregation sowie die Metastasenwirkung inhibierende Mittel angewandt werden. In vivo-Anwendungen umfassen die Verabreichung an Menschen und Tieren, um die Bildung von venösen und arteriellen Blutgerinnseln zu verhindern, wie zur Verhinderung von vorübergehenden ischämischen Anfällen und bei der Langzeitprophylaxe nach Herzinfarkten und Schlaganfällen, sowie bei Arteriosklerose, aber auch bei der Operationsnachbehandlung zur Verhinderung postoperativer Thrombosen sowie bei der Nachbehandlung von Krebs zur Verhinderung bzw. Verminderung der Metastasenwirkung. Auch die Anwendung bei Patienten, die an Herz-Lungen-Maschinen oder an die Nierendialyse angeschlossen sind, ist möglich, ebenso bei Patienten mit künstlichen Herzklappen, Gefäßprothesen, usw. Auch die Anwendung für die eigentlichen Indikationen der Einzelbestandteile, z. B. Verbesserung der Sauerstoffversorgung des Herzmuskels bei Angina pectoris sowie zur Schmerzlinderung und zur Entzündungshemmung ist möglich.

**In vivo Untersuchungen**

Es wurde die Kombination von Dipyridamol oder Mopidamol und Acetylsalicylsäure in vivo mit Hilfe einer Versuchsanordnung bewertet, bei der eine intravaskuläre Thrombose mit einem Laser in den Arteriolen oder Venolen des Mesenteriums einer Ratte erzeugt wurde. Die Auswertung erfolgte mittels vitalmikroskopischer Analyse (Nature, 218, (1968) 887 und Haemostasis 13 (1983) 61 und IRCS Med. Sci. 12 (1984) 91).

Die Testsubstanzen wurden in 0,9 % Natriumchlorid-Lösung (enthielt 1 % Carboxymethylzellulose (Serva, Heidelberg)) appliziert, und zwar entweder per os, intraperitoneal oder intravenös. Kontrolltiere wurden in entsprechender Weise, aber ohne Testsubstanzen behandelt. Als Versuchstiere wurden männliche oder weibliche Sprague Dawley- oder Wistar-Ratten verwendet (Körpergewicht von ca. 200 g). Die zu untersuchenden Tiere wurden mit 0,1 mg Atropinum sulf. sol. s. c. prämediziert und mit 100 mg Ketaminhydrochlorid und 4 mg Xylazin pro kg KG i. p. anästhetisiert. Zur Untersuchung dienten Arteriolen des mit entgastem Paraffinöl überschichteten Mesenteriums mit einem Durchmesser von ca. 13 µm. Der Strahl des 4 W Argon-Lasers (Fa. Spectra Physics, Darmstadt) wurde mittels einer Strahladaptions- und -justieranlage (Fa. BTG, München) koaxial in den invertierten Strahlengang eines Mikroskopes (ICH 405, LD-Epipland 40/0.60; Fa. Zeiss, Oberkochen, Deutschland) eingebracht. Die benutzte Wellenlänge beträgt 514,5 nm mit einer Energie oberhalb des Objektives von 30,5 mW. Die Expositionszeit pro Einzelschuß dauert 1/15 sec. Alle Meßvorgänge wurden per Videokamera (Trinicon-Röhre, Sony, Köln) aufgenommen und auf einem Rekorder (Sony, U-matic 3/4") gespeichert.

Die Testsubstanzen wurden den Versuchstieren in verschiedenen Dosierungen oral eine Stunde, bei i. v. Gabe 10 min vor Versuchsbeginn verabreicht. Kontrolltiere erhielten die gleiche Menge des Placebos. Die Applikation der Substanzen erfolgte 1) als Einzelgabe, 2) zusammen als Kombination oder 3) zuerst Acetylsalicylsäure und nach 1 h Dipyridamol oder Mopidamol sowie 4) zuerst Dipyridamol oder Mopidamol und nach 1 h Acetylsalicylsäure (Tab. 1). Tab. 2 zeigt den Einfluß verschiedener Zeitintervalle.

**Auswertung:**

Es wird die Zahl der Schüsse gezählt, um einen definierten Thrombus zu induzieren. Die Schußfrequenz beträgt eine

Läsion im Abstand von 2 min, wobei alle während des Beobachtungszeitraumes gebildeten Thromben von einer Mindestgröße von 1/4 Gefäßradius ausgezählt und vermessen wurden.

Die statistische Auswertung der Versuchsergebnisse erfolgte mit Hilfe des $\chi^2$-Testes (L. Cavalli-Sforza, Biometrie. Stuttgart, 1969, S. 49 ff).

**Ergebnisse:**

Die Ergebnisse sind in Tabelle 1 dokumentiert. Die Einzelgaben von 5 mg/kg per os von Acetylsalicylsäure oder Dipyridamol bzw. Mopidamol waren nicht signifikant wirksam. Auch die gleichzeitige Verabreichung von je 5 mg/kg Dipyridamol und Acetylsalicylsäure ergab keine antithrombotische Wirkung im Lasermodell. Dies war auch der Fall, wenn zuerst Acetylsalicylsäure und nach 1 h Dipyridamol verabreicht wurde. Hingegen besaß die Anwendung von zuerst Dipyridamol und nach 1 h Acetylsalicylsäure (je 5 mg/kg) eine dosisbezogene, signifikante Wirkung an Arteriolen und Venolen. Der überadditive Effekt dieser zeitlich abgestuften Applikation im Vergleich zur Einzelgabe dokumentiert sich deutlich in der prozentualen Veränderung im Hinblick auf die Kontrolle (Tab. 1) und konnte durch chronische Langzeitgabe (1 Woche) noch gesteigert werden. Verglichen damit konnte durch Anwendung einer Kombination, bestehend aus Dipyridamol und einem hohen Acetylsalicylsäure-Anteil keine Steigerung der antithrombotischen Wirkung mehr erzielt werden, und zwar unabhängig davon, ob gleichzeitig oder in erfindungsgemäßer Reihenfolge appliziert wurde.

Die in Tab. 2 aufgelisteten Ergebnisse zeigen die optimale Zeitspanne zwischen den beiden Einzelgaben.

Die zeitlich abgestufte Anwendung kann mit Hilfe einer handelsüblichen Perfusoreinheit mit zwei getrennt steuerbaren Kammern (z. B. der Fa. Braun, Melsungen, Deutschland mit über Zeitschaltuhr getrennt geschaltetem Motorvorschub) durchgeführt werden. Die beiden Kammern der Perfusoreinheit wurden jeweils mit Dipyridamol-Lösung (entsprechend 5 mg Dipyridamol/kg Ratte) bzw. mit Acetylsalicylsäure-Lösung (entsprechend 5 mg/kg) gefüllt (NaCl-Lösung wie oben). 20 min nach der Injektion der Dipyridamol-Lösung in die Schwanzvene erfolgte die durch Schaltuhr gesteuerte Injektion der Acetylsalicylsäure-Lösung. Im Vergleichsexperiment wurden die beiden Kammern gleichzeitig injiziert. Die Ergebnisse entsprachen den nach oraler Gabe erhaltenen Meßwerten, d. h. durch die zeitlich abgestufte Anwendung wurden Wirkungen erhalten, die weit über den bei gleichzeitiger Verabreichung erhaltenen lagen.

**Pharmazeutische Zubereitungen**

Zur Erzielung der überadditiven Effekte können auch Suspensionen und feste Zubereitungen, die für die orale, perorale und rektale Applikation geeignet sind, verwendet werden. Beispiele solcher Zubereitungen für die Anwendung am Menschen enthalten x mg Dipyridamol und/oder Mopidamol als Reinsubstanz oder als handelsübliche Fertigzubereitungen (Persantin® Dragees oder Ampullenlösung der Fa. Dr. Karl Thomae GmbH, Biberach, Deutschland) oder Anteile dieser Fertigzubereitungen, kombiniert mit y mg Acetylsalicylsäure in Form von im Handel erhältlichen Mikrokapseln (-granulaten) (Typ M80D der Fa. Röhm Pharma GmbH, Weiterstadt, Deutschland, Encaprin®, Procter & Gamble, Connecticut, USA). Die pharmazeutischen Träger dieser Kombinationen sind durch Erhitzen verfestigte Gele aus
a) 20 Gewichtsprozent Gelatine / 1 Gewichtsprozent Glycerin in Wasser sowie
b) 1 Gewichtsprozent Agarose in Wasser sowie
c) 10 Gewichtsprozent Äthylcellulose T50 (Hercules GmbH, Hamburg) in Aceton/Wasser (Gewichtsverhältnis 80 : 20),
jeweils mit 8 Gewichtsprozent eingerührtem Dipyridamol oder Mopidamol oder aber handelsübliche Gelatinekapseln (für die Anwendung am Menschen und Großtieren vorzugsweise Größe O (Fa. Kapsugel, Basel).

Die pharmazeutischen Zubereitungen (siehe Tabelle 3, Beispiele 1 - 6) werden in 10 ml Magensaft des Hundes bzw. 10 ml 0,1 N HCl eingegeben und unter schonendem Rühren bei 37°C gehalten. Aliquote des Überstandes werden zu gewissen Zeitintervallen entnommen und mittels Hochdruckflüssigkeitschromatographie analog der Vorschrift in Journal of Chromatography <u>231</u> (1982) 216 - 221) aufgetrennt und quantifiziert. In analoger Weise werden die pharmazeutischen Zubereitungen in Duodenalflüssigkeit (Hund) bzw. Natriumhydrogencarbonatlösung (pH = 7,4) eingesetzt.

**In vivo-Versuche an der Ratte**

Für die Anwendung werden die Bestandteile der in den Beispielen 1 - 6 genannten Zubereitungen jeweils auf die in Tabelle 1 beschriebenen Wirkstoffmengen reduziert bzw. Kapseln der Größe 4 und 5 verwendet. Tabelle 1 zeigt, daß mit den erfindungsgemäßen Präparaten X, XI und XIII ein überadditiver stärkerer antithrombotischer Effekt erzielt wurde der weit höher war als bei gleichzeitiger Verabreichung der gleichen Mengen beider Komponenten. Damit wird bei sogar verbesserter Wirkung eine erhebliche Verminderung der verabreichten Acetylsalicylsalicylsäure und auch des verabreichten Pyrimido-pyrimidins und damit eine erheblich niedrigere Belastung des Körpers möglich.

**Tabelle 1**

Wirkung von Pyrimido-pyrimidin und/oder Acetylsalicylsäure bei verschiedener Applikationsfolge auf die Laser-induzierte Thrombose an der Ratte

| Versuch | Substanz | Dosis mg/kg KG p. o. | Anzahl Tiere n | Anzahl der Läsionen/ Tier | Anzahl der Schüsse x | SEM | Veränderungen gegen Kontrolle absolut | % | $\chi^2$-Test |
|---|---|---|---|---|---|---|---|---|---|
| I | Kontrolle (Placebo) | – | 12 | 48 | 2,17 | 0,01 | — | — | |
| II | Dipyridamol | 5 | 6 | 24 | 2,35 | 0,14 | 0,18 | 8 | |
| III | Mopidamol | 5 | 6 | 24 | 2,50 | 0,19 | 0,33 | 15 | |
| IV | Acetylsalicylsäure | 5 | 6 | 24 | 1,79 | 0,20 | -0,38 | -18 | |
| V | Acetylsalicylsäure | 10 | 6 | 24 | 2,92 | 0,20 | 0,75 | 35 | p< 0.01 |
| VI | Acetylsalicylsäure | 22 | 6 | 24 | 2,99 | 0,17 | 0,82 | 38 | p< 0.01 |
| VII | Dipyridamol + gleichzeitig Acetylsalicylsäure | 5 +5 | 6 | 24 | 2,25 | 0,19 | 0,08 | 4 | |
| VIII | Dipyridamol + gleichzeitig Acetylsalicylsäure | 5 +10 | 6 | 24 | 2,82 | 0,21 | 0,65 | 30 | |
| IX | Dipyridamol + gleichzeitig Acetylsalicylsäure | 5 +22 | 6 | 24 | 3,27 | 0,22 | 1,10 | 51 | p< 0.01 |
| X | Dipyridamol; nach 1 h Acetylsalicylsäure | 5 +5 | 6 | 24 | 3,27 | 0,20 | 1,10 | 51 | p< 0.01 |
| XI | Dipyridamol; nach 1 h Acetylsalicylsäure | 5 +9,8 | 6 | 24 | 3,10 | 0,18 | 0,93 | 43 | p< 0.01 |
| XII | Dipyridamol; nach 1 h Acetylsalicylsäure | 5 +22 | 6 | 24 | 3,26 | 0,16 | 1,09 | 50 | p< 0.01 |
| XIII | Mopidamol; nach 1 h Acetylsalicylsäure | 5 +5 | 6 | 24 | 2,82 | 0,17 | 0,65 | 30 | p< 0.01 |

**Tab. 2**

Wirkung von Pyrimido-pyrimidin und Acetylsailcylsäure auf die Laser-induzierte Thrombose an der Ratte. Einfluß des Zeitintervalls zwischen der Verabreichung der Substanzen. Dosierung jeweils 5 mg/kg Dipyridamol p. o., dann nach der angegebenen Zeit 5 mg/kg Acetylsalicylsäure p. o.

| Substanz | Anzahl der Tiere | Anzahl der Läsionen | $\bar{x}$ | SEM | % Veränderung gegen Kontrolle | $\chi^2$-Test |
|---|---|---|---|---|---|---|
| Kontrolle (Placebo) | 12 | 48 | 2,17 | 0,01 | – | |
| 0 min | 6 | 24 | 2,25 | 0,19 | 4 | |
| 10 min | 6 | 24 | 2,27 | 0,22 | 5 | |
| 20 min | 6 | 24 | 2,56 | 0,18 | 18 | |
| 30 min | 6 | 24 | 3,23 | 0,21 | 49 | p< 0.01 |
| 60 min | 6 | 24 | 3,27 | 0,20 | 51 | p< 0.01 |
| 90 min | 4 | 16 | 3,05 | 0,22 | 41 | p< 0.01 |
| 120 min | 4 | 16 | 2,30 | 0,25 | 6 | |

**Tabelle 3**

| Beispiel | Pharmazeutischer Träger | Acetylsalicylsäure-Gehalt | Pyrimidopyrimidin-Gehalt | % Acetylsalicylsäure freigesetzt nach (...min) bei pH=1,8 | % Pyrimidopyrimidin |
|---|---|---|---|---|---|
| 1 | Kapsel | 103 mg (M80D) | 75 mg Dipyridamol | 0 (5) | 49 (5) |
| | | | | 0 (10) | 89 (10) |
| | | | | 0 (30) x) | 100 (30) |
| 2 | Kapsel | 97 mg (M80D) | 150 mg Mopidamol | 0 (5) | 45 (5) |
| | | | | 0 (10) | 87 (10) |
| | | | | 0 (30) x) | 100 (30) |
| 3 | Gelatine | 80 mg (M80D) | 50 mg Dipyridamol | 0 (5) | 88 (5) |

| | | | | | |
|---|---|---|---|---|---|
| | | | | 0 (10)<br>0 (30) x) | 100 (10)<br>100 (30) |
| 4 | wie 3 | 45 mg (M80D) | 69 mg Dipyridamol | 0 (5)<br>0 (10)<br>0 (30) x) | 85 (5)<br>100 (10)<br>100 (30) |
| 5 | Agarose | 138 mg (M80D) | 150 mg Dipyridamol | 0 (5)<br>0 (10)<br>0 (30) x) | 36 (5)<br>76 (10)<br>100 (30) |
| 6 | Suspension aus 4 ml Dipyridamol-Hydrochlorid-Lsg. und M80D | 20 mg (M80D) | 20 mg Dipyridamol | 0 (5)<br>0 (10)<br>0 (30) x) | 100 (5)<br>100 (10)<br>100 (30) |

x) Die magensäreunlöslichen Acetylsalicylsäure-Kristalle M80D werden bei pH = 7,4 quantitativ gelöst.

**Formelblatt**

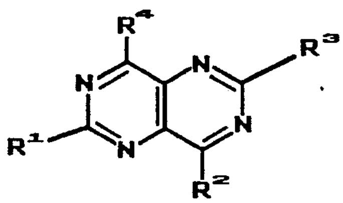

Dipyridamol (I a) $R^1 = R^3$ = - N $(CH_2\text{-}CH_2\text{-}OH)_2$

$R^2 = R^4$ = - 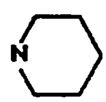

Mopidamol (I b) $R^1 = R^3$ = - N $(CH_2\text{-}CH_2\text{-}OH)_2$

$R^2$ = - H

$R^4$ = - 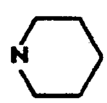

I c: $R^1$ und $R^2$ = H $R^3$ = - N $(CH_2\text{-}CH_2\text{-}OH)_2$

$R^4$ = - 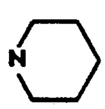

I d: $R^1$ und $R^4$ = H $R^2$ = - 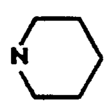

$R^3$ = - N $(CH_2\text{-}CH_2\text{-}OH)_2$

**Patentansprüche** for the Contracting States: BE, CH, DE, FR, GB, IT, LI, LU, NL, SE

1. Pharmazeutische Zubereitung, enthaltend
A) einerseits ein Pyrimido-pyrimidin der Formel I,

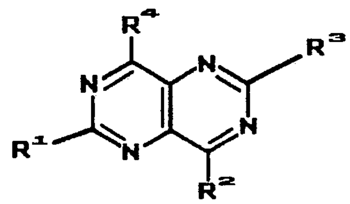

(I)

worin wenigestens einer der Reste $R^1$ und $R^3$ den Rest $-N(CH_2-CHR^5-OH)_2$ mit $R^5$ = Wasserstoff oder Methyl und wenigstens einer der Reste $R^2$ und $R^4$ den Rest

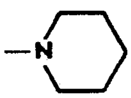

darstellt, der in p-Stellung zum Stickstoffatom auch durch Sauerstoff unterbrochen sein kann, bzw. dessen wirksame Metabolite und/oder Salze und
B) andererseits O-Acetylsalicylsäure bzw. deren pharmazeutisch vertragliche Salze, wobei das Gewichtsverhältnis der Komponente A) zur Komponente B) größer als 0,5 ist, mit oder ohne
C) einen pharmazeutischen Träger zur Anwendung in der Therapie von Krankheiten, die durch gestörte Blutfunktionen bzw. Blutbestandteile, insbesondere Thrombocyten bzw. Erythrocyten verursacht bzw. gekennzeichnet sind, derart formuliert, daß die Komponente A) zuerst von der Zubereitung freigesetzt wird, und die Komponente B) nach einem Zeitabstand von 15 Minuten bis 2 Stunden danach von der Zubereitung freigesetzt wird.

2. Ausführungsform nach Anspruch 1, dadurch gekennzeichnet, daß die Komponente A) in Form des Dipyridamols vorliegt.

3. Ausführungsform nach Anspruch 1 oder 2, dadurch gekennzeichnet, daß die Zubereitung in einer oral oder rektal zu verabreichenden Dosiereinheit vorliegt, vorzugsweise einer solchen, die 10 bis 150, vorzugsweise 25 bis 75 mg Dipyridamol und 10 bis unter 300 mg Acetylsalicylsäure-Komponente bzw. 100 bis 600, vorzugsweise 200 bis 250 mg Mopidamol und 10 bis unter 1200, vorzugsweise 100 bis 500 mg Acetylsalicylsäure-Komponente enthält.

4. Ausführungsform nach einem oder mehreren der Ansprüche 1 bis 3, dadurch gekennzeichnet, daß der obere Grenzwert des Gewichtsverhältnisses der Komponente A) zur Komponente B) bei 30, vorzugsweise bei 10 und insbesondere bei 3 liegt und der untere Grenzwert vorzugsweise 0,6 ist.

5. Ausführungsform nach einem oder mehreren der Ansprüche 1 bis 4, dadurch gekennzeichnet, daß das Trägermaterial für die Acetylsalicylsäre-Komponente wenigstens ein Retardierungsmittel enthält.

6. Ausführungsform nach einem oder mehreren der Ansprüche 1 bis 5, dadurch gekennzeichnet, daß die Zubereitung in Form von Mikrokapseln vorliegt, wobei das Kapselmaterial für die Acetylsalicylsäure-Komponente magensaftresistent ist oder eine verzögerte Freisetzung ermöglicht.

7. Ausführungsform nach einem oder mehreren der Ansprüche 1 bis 6, dadurch gekennzeichnet, daß die Zubereitung in Form einer Mantel- oder Schichttablette vorliegt, in der die Primido-pyrimidin-Komponente in der schneller zu resorbierenden Schicht enthalten ist.

8. Ausführungsform nach einem oder mehreren der Ansprüche 1 bis 6, dadurch gekennzeichnet, daß das Pyrimido-pyrimidin A) und die Acetylsalicylsäre-Komponente B) - letztere in entsprechend verzögerter Form - in voneinander getrennten Dosiseinheiten zur gleichzeitigen Verabreichung vorliegen.

9. Verfahren zur Herstellung von pharmazeutischen Zubereitungen, dadurch gekennzeichnet, daß man
A) ein Pyrimido-pyrimidin der Formel I gemäß Anspruch 1 bzw. dessen wirksame Metabolite und/oder Salze und
B) O-Acetylsalicylsäure bzw. deren pharmazeutisch verträgliche Salze,
C) mit oder ohne pharmazeutische Träger in üblicher Weise zu Mantel- oder Schichttabletten oder Zäpfchen mit der Pyrimido-pyrimidin-Komponente A) in der äußeren, insbesondere magensaftlöslichen Schicht und der Komponente B) im Kern bzw. der anderen Schicht in einer weniger magensaftlöslichen, jedoch darmasaftlöslichen Form, verarbeitet oder als Kapsel formuliert, derart, daß das Gewichtsverhältnis der Komponente A) zur Komponente B) größer als 0,5 ist

und daß die Komponente A) zuerst von der Zubereitung freigesetzt wird und die Komponente B) nach einem Zeitabstand von 15 Minuten bis 2 Stunden danach von der Zubereitung freigesetzt wird.

**Patentansprüche** for the Contracting State: AT

1. Verfahren zur Herstellung von pharmazeutischen Zubereitungen, dadurch gekennzeichnet, daß man
A) ein Pyrimido-pyrimidin der Formel I,

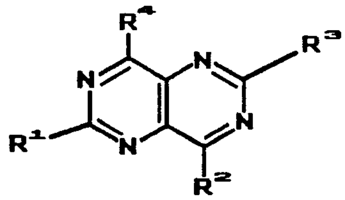

(I)

worin wenigestens einer der Reste $R^1$ und $R^3$ den Rest $-N(CH_2-CHR^5-OH)_2$ mit $R^5$ = Wasserstoff oder Methyl und wenigstens einer der Reste $R^2$ und $R^4$ den Rest

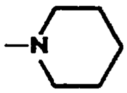

darstellt, der in p-Stellung zum Stickstoffatom auch durch Sauerstoff unterbrochen sein kann, bzw. dessen wirksame Metabolite und/oder Salze und
B) andererseits O-Acetylsalicylsäure bzw. deren pharmazeutisch verträgliche Salze,
C) mit oder ohne pharmazeutischen Träger in üblicher Weise zu Mantel- oder Schichttabletten oder Zäpfchen mit der Pyrimido-pyrimidin-Komponente A) in der äußeren, insbesondere magensaftlöslichen Schicht und der Komponente B) im Kern bzw. der anderen Schicht in einer weniger magensaftlöslichen, jedoch darmsaftlöslichen Form, verarbeitet oder als Kapsel(n) formuliert derart, daß das Gewichtsverhältnis der Komponente A) zur Komponente B) größer als 0,5 ist, und daß die Komponente A) zuerst von der Zubereitung freigesetzt wird und die Komponente B) nach einem Zeitabstand von 15 Minuten bis 2 Stunden danach von der Zubereitung freigesetzt wird.

2. Verfahren nach Anspruch 1, dadurch gekennzeichnet, daß die Komponente A) in Form des Dipyridamols vorliegt.

3. Verfahren nach Anspruch 1 oder 2, dadurch gekennzeichnet, daß die Zubereitung in einer oral oder rektal zu verabreichenden Dosiereinheit vorliegt, vorzugsweise einer solchen, die 10 bis 150, vorzugsweise 25 bis 75 mg Dipyridamol und 10 bis unter 300 mg Acetylsalicylsäure-Komponente bzw. 100 bis 600, vorzugsweise 200 bis 250 mg Mopidamol und 10 bis unter 1200, vorzugsweise 100 bis 500 mg Acetylsalicylsäure-Komponente enthält.

4. Verfahren nach einem oder mehreren der Ansprüche 1 bis 3, dadurch gekennzeichnet, daß der obere Grenzwert des Gewichtsverhältnisses der Komponente A) zur Komponente B) bei 30, vorzugsweise bei 10 und insbesondere bei 3 liegt und der untere Grenzwert vorzugsweise 0,6 ist.

5. Verfahren nach einem oder mehreren der Ansprüche 1 bis 4, dadurch gekennzeichnet, daß das Trägermaterial für die Acetylsalicysäre-Komponente wenigstens ein Retardierungsmittel enthält.

6. Verfahren nach einem oder mehreren der Ansprüche 1 bis 5, dadurch gekennzeichnet, daß die Zubereitung in Form von Mikrokapseln vorliegt, wobei das Kapselmaterial für die Acetylsalicylsäure-Komponente magensaftresistent ist oder eine verzögerte Freisetzung ermöglicht.

7. Verfahren nach einem oder mehreren der Ansprüche 1 bis 6, dadurch gekennzeichnet, daß die Zubereitung in Form einer Mantel- oder Schichttablette vorliegt, in der die Pyrimido-pyrimidin-Komponente in der schneller zu resorbierenden Schicht enthalten ist.

8. Verfahren nach einem oder mehreren der Ansprüche 1 bis 6, dadurch gekennzeichnet, daß das Pyrimido-pyrimidin A) und die Acetylsalicylsäure-Komponente B) - letztere in entsprechend verzögerter Form - in voneinander getrennten Dosiseinheiten zur gleichzeitigen Verabreichung vorliegen.

**Claims** for the Contracting States: BE, CH, DE, FR, GB, IT, LI, LU, NL, SE

1. A pharmaceutical formulation containing
A) on the one hand a pyrimido-pyrimidine of the formula I

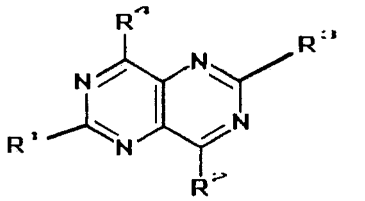

(I)

in which at least one of the radicals $R^1$ and $R^3$ represents the radical $-N(CH_2-CHR^5-OH)_2$ where $R^5$ = hydrogen or methyl, and at least one of the radicals $R^2$ and $R^4$ represents the radical

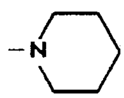

which can also be interrupted by oxygen in the p-position relative to the nitrogen atom, or active metabolites and/or salts thereof, and
B) on the other hand O-acetylsalicylic acid or pharmaceutically tolerated salts thereof, the weight ratio of component A) to component B) being greater than 0.5, with or without
C) a pharmaceutical vehicle, for administration in the therapy of diseases caused or characterized by impaired blood functions or constituents of blood, in particular platelets or erythrocytes, formulated such that component A) is released first from the formulation, and component B) is released from the formulation after an interval of 15 minutes to 2 hours thereafter.

2. The embodiment as claimed in claim 1, wherein component A) is in the form of dipyridamol.

3. The embodiment as claimed in either of claims 1 and 2, wherein the formulation is in the form of a dosage unit for oral or rectal administration, preferably one which contains 10 to 150, preferably 25 to 75, mg of dipyridamol and 10 to less than 300 mg of acetylsalicylic acid component or 100 to 600, preferably 200 to 250, mg of mopidamol and 10 to less than 1,200, preferably 100 to 500, mg of acetylsalicylic acid component.

4. The embodiment as claimed in one or more of claims 1 to 3, wherein the upper limit for the ratio by weight of component A) to component B) is 30, preferably 10, and in particular 3, and the lower limit is preferably 0.6.

5. The embodiment as claimed in one or more of claims 1 to 4, wherein the vehicle for the acetylsalicylic acid component contains at least one retarding agent.

6. The embodiment as claimed in one or more of claims 1 to 5, wherein the formulation is in the form of microcapsules, the capsule material for the acetylsalicylic acid component being resistant to gastric juice or permitting delayed release.

7. The embodiment as claimed in one or more of claims 1 to 6, wherein the formulation is in the form of a coated or layered tablet in which the pyrimido-pyrimidine component is contained in the layer which is to be absorbed more rapidly.

8. The embodiment as claimed in one or more of claims 1 to 6, wherein the pyrimidopyrimidine A) and the acetylsalicylic acid component B) - the latter in appropriately delayed form - are present in dose units, which are separated from one another, for simultaneous administration.

9. A process for the preparation of pharmaceutical formulations, which comprises processing
A) a pyrimidopyrimidine of the formula I as claimed in claim 1, or its active metabolites and/or salts, and
B) O-acetylsalicylic acid, or its pharmaceutically tolerated salts,
C) with or without pharmaceutical vehicles, in a customary manner to give coated or layered tablets or suppositories with the pyrimidopyrimidine component A) in the outer layer which is, in particular, soluble in gastric juice, and the component B) in the core or the other layer in a form which is less soluble in gastric juice but is soluble in intestinal juice, or formulating them as capsules in such a manner that the ratio by weight of component A) to component B) is greater than 0.5 and that component A) is released first from the formulation and component B) is released from the formulation after an interval of 15 minutes to 2 hours thereafter.

**Claims** for the Contracting State: AT

1. A process for the preparation of pharmaceutical formulations, which comprises processing A) a pyrimidopyrimidine of the formula I

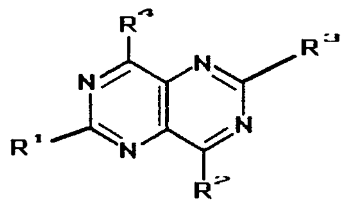

(I)

in which at least one of the radicals $R^1$ and $R^3$ represents the radical $-N(CH_2-CHR^5-OH)_2$ where $R^5$ = hydrogen or methyl, and at least one of the radicals $R^2$ and $R^4$ represents the radical

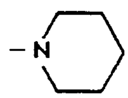

which can also be interrupted by oxygen in the p-position relative to the nitrogen atom, or active metabolites and/or salts thereof, and
B) on the other hand O-acetylsalicylic acid or pharmaceutically tolerated salts thereof,
C) with or without a pharmaceutical vehicle, in a customary manner to give coated or layered tablets or suppositories with the pyrimidopyrimidine component A) in the outer layer which is, in particular, soluble in gastric juice, and the component B) in the core or the other layer in a form which is less soluble in gastric juice but is soluble in intestinal juice, or formulating them as capsule(s) in such a manner that the ratio by weight of component A) to component B) is greater than 0.5 and that component A) is released first from the formulation and component B) is released from the formulation after an interval of 15 minutes to 2 hours therafter.

2. The process as claimed in claim 1, wherein component A) is in the form of dipyridamol.

3. The process as claimed in either of claims 1 and 2, wherein the formulation is in the form of a dosage unit for oral or rectal administration, preferably one which contains 10 to 150, preferably 25 to 75, mg of dipyridamol and 10 to less than 300 mg of acetylsalicylic acid component or 100 to 600, preferably 200 to 250, mg of mopidamol and 10 to less than 1.200, preferably 100 to 500, mg of acetylsalicylic acid component.

4. The process as claimed in one or more of claims 1 to 3, wherein the upper limit for the ratio by weight of component A) to component B) is 30, preferbly 10, and in particular 3, and the lower limit is preferably 0.6.

5. The process as claimed in one or more of claims 1 to 4, wherein the vehicle for the acetylsalicylic acid component contains at least one retarding agent.

6. The process as claimed in one or more of claims 1 to 5, wherein the formulation is in the form of microcapsules, the capsule material for the acetylsalicylic acid component being resistant to gastric juice or permitting delayed release.

7. The process as claimed in one or more of claims 1 to 6, wherein the formulation is in the form of a coated or layered tablet in which the pyrimido-pyrimidine component is contained in the layer which is to be absorbed more rapidly.

8. The process as claimed in one or more of claims 1 to 6, wherein the pyrimidopyrimidine A) and the acetylsalicylic acid component B) - the latter in appropriately delayed form - are present in dose units, which are separated from one another, for simultaneous administration.

**Revendications** pour les Etats Contractants: BE, CH, DE, FR, GB, IT, LI, LU, NL, SE

1. Composition pharmaceutique contenant, d'une part,
A) une pyrimido-pyrimidine de formule I

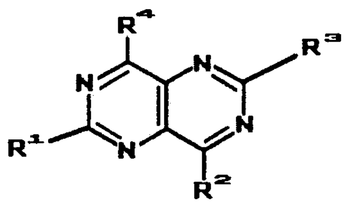

(I)

dans laquelle au moins l'un des radicaux $R^1$ et $R^3$ représente le radical $-N(CH_2-CHR^5-OH)_2$, $R^5$ étant un atome d'hydrogène ou le groupe méthyle, et au moins l'un des radicaux $R^2$ et $R^4$ représente le radical

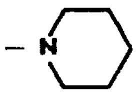

qui peut également être interrompu par un atome d'oxygène en position para par rapport à l'atome d'azote, ou ses métabolites actifs et/ou ses sels, et d'autre part
B) de l'acide O-acétylsalicylique ou ses sels pharmaceutiquement acceptables, le rapport pondéral du composant A) au composant B) étant supérieur à 0,5, avec ou sans
C) un véhicule pharmaceutique pour utilisation dans le traitement de maladies qui sont causées ou caractérisées par des fonctions sanguines perturbées ou des constituants sanguins perturbés, en particulier les trombocytes ou les érythrocytes, formulée de telle façon que le composant A) est d'abord libéré de la composition et le composant B), après un intervalle de 15 minutes à 2 heures, est ensuite libéré de la composition.

2. Mode de réalisation selon la revendication 1, caractérisé en ce que le composant A) se trouve sous forme du dipyridamole.

3. Mode de réalisation selon la revendication 1 ou 2, caractérisé en ce que la composition se trouve en une dose unitaire à administrer par voie orale ou rectale, de préférence en une dose unitaire qui contient de 10 à 150, de préférence de 25 à 75 mg de dipyridamole, et de 10 à moins de 300 mg, de composant acide acétylsalicylique, ou de 100 à 600, de préférence de 200 à 250 mg de mopidamole et de 10 à moins de 1 200, de préférence de 100 à 500 mg de composant acide acétylsalicylique.

4. Mode de réalisation selon une ou plusieurs des revendications 1 à 3, caractérisé en ce que la limite supérieure du rapport pondéral du composant A) au composant B) se situe à 30, de préférence à 10 et en particulier à 3, et la limite inférieure est de préférence 0,6.

5. Mode de réalisation selon une ou plusieurs des revendications 1 à 4, caractérisé en ce que le véhicule pour le composant acide acétylsalicylique contient au moins un agent de retardement.

6. Mode de réalisation selon une ou plusieurs des revendications 1 à 5, caractérisé en ce que la composition se trouve sous forme de microcapsules, le matériau des capsules pour le composant acide acétylsalicylique étant résistant au suc gastrique ou permettant une libération retardée.

7. Mode de réalisation selon une ou plusieurs des revendications 1 à 6, caractérisé en ce que la composition se trouve sous forme d'un comprimé stratifié ou enrobé, dans lequel le composant pyrimido-pyrimidine est contenu dans la couche la plus rapide à se résorber.

8. Mode de réalisation selon une ou plusieurs des revendications 1 à 6, caractérisé en ce que la pyrimidopyrimidine A) et le composant acide acétylsalicylique B) - ce dernier sous forme retard appropriée - se trouvent en doses unitaires séparées l'une de l'autre, pour l'administration simultanée.

9. Procédé pour la fabrication de compositions pharmaceutiques, caractérisé en ce que l'on transforme A) une pyrimido-pyrimidine de formule I selon la revendication 1 et/ou ses métabolites actifs et/ou sels, et
B) de l'acide O-acétylsalicylique ou ses sels pharmaceutiquement acceptables,
C) avec ou sans véhicule pharmaceutique, de façon usuelle, en comprimés enrobés ou stratifiés, ou en suppositoires, le composant pyrimido-pyrimidine A) étant dans la couche externe en particulier soluble dans le suc gastrique, et le composant B) étant dans le noyau ou dans l'autre couche, sous une forme moins soluble dans le suc gastrique mais soluble dans le suc intestinal, ou on les formule en capsule, de manière que le rapport pondéral du composant A) au composant B) soit supérieur à 0,5 et que le composant A) soit libéré en premier de la composition et que le composant B) soit libéré ensuite de la composition, après un intervalle de 15 minutes à 2 heures.

**Revendications** pour l'Etat Contractant: AT

1. Procédé pour la préparation de compositions pharmaceutiques, caractérisé en ce que l'on transforme
A) une pyrimido-pyrimidine de formule I

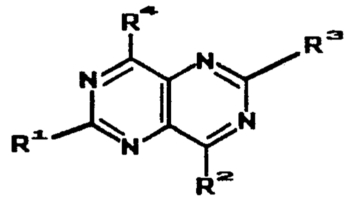

(I)

dans laquelle au moins l'un des radicaux $R^1$ et $R^3$ représente le radical $-N(CH_2-CHR^5-OH)_2$ $R^5$ étant un atome d'hydrogène ou le groupe méthyle, et au moins l'un des radicaux $R^2$ et $R^4$ représente le radical

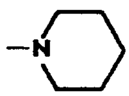

qui peut également être interrompu par un atome d'oxygène en position para par rapport à l'atome d'azote, ou ses métabolites actifs et/ou ses sels, et d'autre part
B) de l'acide O-acétylsalicylique ou ses sels pharmaceutiquement acceptables,
C) avec ou sans véhicule pharmaceutique, de façon usuelle, en comprimés enrobés ou stratifiés, ou en suppositoires, le composant pyrimido-pyrimidine A) étant dans la couche externe en particulier soluble dans le suc gastrique, et le composant B) étant dans le noyau ou dans l'autre couche, sous une forme moins soluble dans le suc gastrique mais soluble dans le suc intestinal, ou on les formule en capsule(s) de manière que le rapport pondéral du composant A) au composant B) soit supérieur à 0,5 et que le composant A) soit libéré en premier de la composition et que le composant B) soit libéré ensuite de la composition, après un intervalle de 15 minutes à 2 heures.

2. Procédé selon la revendication 1, caractérisé en ce que le composant A) se trouve sous forme du dipyridamole.

3. Procédé selon la revendication 1 ou 2, caractérisé en ce que la composition se trouve en une dose unitaire à administrer par voie orale ou rectale, de préférence en une dose unitaire qui contient de 10 à 150, de préférence de 25 à 75 mg de dipyridamole, et de 10 à moins de 300 mg de composant acide acétylsalicylique, ou de 100 à 600 de préférence de 200 à 250 mg de mopidamole et de 10 à moins de 1 200, de préférence de 100 à 500 mg de composant acide acétylsalicylique.

4. Procédé selon une ou plusieurs des revendications 1 à 3, caractérisé en ce que la limite supérieure du rapport pondéral du composant A) au composant B) se situe à 30, de préférence à 10 et en particulier à 3, et la limite inférieure est de préférence 0,6.

5. Procédé selon une ou plusieurs des revendications 1 à 4, caractérisé en ce que le véhicule pour le composant acide acétylsalicylique contient au moins un agent de retardement.

6. Procédé selon une ou plusieurs des revendications 1 à 5, caractérisé en ce que la composition se trouve sous forme de microcapsules, le matériau des capsules pour le composant acide acétylsalicylique étant résistant au suc gastrique ou permettant une libération retardée.

7. Procédé selon une ou plusieurs des revendications 1 à 6, caractérisé en ce que la composition se trouve sous forme d'un comprimé stratifié ou enrobé, dans lequel le composant pyrimido-pyrimidine est contenu dans la couche la plus rapide à se résorber.

8. Procédé selon une ou plusieurs des revendications 1 à 6, caractérisé en ce que la pyrimidopyrimidine A) et le composant acide acétylsalicylique B) - ce dernier sous forme retard appropriée - se trouvent en doses unitaires séparées l'une de l'autre, pour l'administration simultanée.